# EUROPÄISCHE PATENTSCHRIFT

(11) **EP 0 975 320 B1**
(45) Veröffentlichungstag und Bekanntmachung des Hinweises auf die Patenterteilung: **19.12.2001**
(21) Anmeldenummer: 98912244.5
(22) Anmeldetag: 06.02.1998
(51) Int. Cl.: A61K 7/48

(54) **FESTE KOSMETISCHE ZUSAMMENSETZUNG AUF BASIS VERFESTIGTER ÖLE UND LANOSTERIN**
SOLID COSMETIC COMPOSITIONS ON THE BASIS OF SOLIDIFIED OILS AND LANOSTERIN
COMPOSITIONS COSMETIQUES SOLIDES A BASE D'HUILES SOLIDIFIEES ET DE LANOSTERINE

(30) Priorität: 11.02.1997 DE 19707309
(43) Veröffentlichungstag der Anmeldung: 02.02.2000
(73) Patentinhaber: LANCASTER GROUP GmbH, 55116 Mainz (DE)
(72) Erfinder: DE CLERMONT-GALLERANDE, Hélène, F-08320 Cap d'Ail (FR); ZASTROW, Leonhard, MC-98000 Monaco (MC); GOLZ-BERNER, Karin, MC-98000 Monaco (MC)
(74) Vertreter: Walter, Wolf-Jürgen
(86) Internationale Anmeldenummer: DE9800449
(87) Internationale Veröffentlichungsnummer: WO9834588

(56) Entgegenhaltungen:
- GB-A- 884 688

## Beschreibung

Die Erfindung betrifft feste kosmetische Zusammensetzungen auf Basis verfestigter öle. Derartige Zusammensetzungen sind infolge ihres wachsartigen Charakters und ihrer Transparenz insbesondere für kosmetische Stifte mit unterschiedlichen Zusatzstoffen geeignet.

Die Basis bisheriger transparenter und fester kosmetischer Formulierungen sind meist Seifen von Fettsäuren, wie Natriumstearat oder Natriumcocoat oder solche mit anderen mittel- bis langkettigen Fettsäureresten, wie Stearyl, Isostearyl, Palmityl oder Talgsäureresten. Als Gegenionen werden zumeist Alkali-Ionen wie Natrium oder Lithium oder auch Triethanolamin eingesetzt. Man erhält dabei feste transparente Formulierungen, die insbesondere als Kernseifen oder in einem Deodorant oder Antitranspirant eingesetzt werden. Bekannt als Handelsprodukt ist beispielsweise Stearinsäuremonoethanolamid.

Weiterhin ist als Basis von Deodorantien oder Antitranspirantien ein Gemisch von Polyolen und Dibenzyliden-ose oder Dibenzyliden-monosorbitol mit Polyolanteilen meist oberhalb von 50 Gew-% bekannt, um einen Gelierungseffekt zu erreichen. Der hohe Polyolanteil zieht jedoch einen hohen transepidermalen Wasserverlust nach sich.

In der EP-A-291331 sind transparente Stifte als Antitranspirant aus Aluminiumchlorhydrat, nichtionischen oberflächenaktiven Mitteln, erweichenden ölen (Emollients) und Wasser beschrieben.

Die EP-A-451002 beschreibt klare Antitranspirant-Stifte, die aus dem Antitranspirant, einem zweiwertigen Alkohol, Dibenzylmonosorbitolacetal, N-Methylpyrrolidon sowie einem Emollient und einem Stabilisator bestehen.

Schließlich wird in der EP-A-665007 die Schwierigkeit der Herstellung transparenter Antitranspirant-Gele geschildert infolge der beschränkten Auswahl an Grundsubstanzen, die überhaupt klare Gele bilden, und eine spezielle Zusammensetzung aus einem spezifischen Siliconöl und 12-Hydroxystearinsäure oder einem Salz davon sowie einem Deodorant beschrieben, bei der alle Bestandteile einen Brechungsindex von 1,48 bis 1,53 cSt haben müssen.

Zum anderen beschreibt die EP-A-636359 eine desodorierende Wirkstoffkombination auf der Basis von alpha-omega-Alkandicarbonsäuren und Wollwachssäuren, die u.a. auch mit den üblichen Hilfsmitteln zu festen Stiften verarbeitet werden kann.

Bei den auf Seifen basierenden Deodorantien sind die aktiven Bestandteile in einem Alkohol gelöst, oder, bei der alkoholfreien Formulierung, in dem Polyol. Der hohe Seifenanteil ist jedoch nicht ohne weiteres auf andere Formulierungen, wie z.B. Deodorants übertragbar.

Aus der GB-A-884688 ist die wahlweise Verwendung von Sterolen im Zusammenhang mit Fettsäuretriglyceriden, Wachsen und C₁₀-C₆₀-Kohlenwasserstoffen als Hautkonditioniermittel bekannt.

Der Erfindung liegt die Aufgabe zugrunde, eine feste kosmetische Zusammensetzung zu entwickeln, die von der kosmetischen Grundlage her ohne Irritationsprobleme für alle Hauttypen anwendbar ist, und die bevorzugt vollständig oder im wesentlichen transparent ist.

Eine speziellere Aufgabe ist die Bereitstellung klarer oder lichtdurchlässiger kosmetischer Stifte mit zusätzlichen kosmetischen Wirk- oder Duftstoffen.

Die neue feste kosmetische Zusammensetzung auf Basis verfestigter öle ist dadurch gekennzeichnet, daß sie 40 bis 95 Gew-% eines kosmetisch annehmbaren öles oder ölgemisches und 1 bis 10 Gew-% Lanosterin enthält und transparent ist, wobei die Anteile jeweils bezogen sind auf die Gesamtmenge der Zusammensetzung.

Ein wesentliches Merkmal der Erfindung besteht darin, daß die Zusammensetzung transparent ist, d.h. vollständig klar und maximal lichtdurchlässig. Andere Ausführungsformen können je nach zugesetzten Stoffen auch transluzent sein, d.h. mit etwas herabgesetzter Lichtdurchlässigkeit aber durchsichtig.

Eine weitere Ausführungsform der Erfindung mit hohen Pigmentanteilen oder Zusatzstoffen, die eine Trübung hervorrufen, kann auch undurchsichtig sein.

Vorteilhaft enthält die erfindungsgemäße Zusammensetzung 0 - 58 Gew-% eines oder mehrerer kosmetischer Wirkstoffe oder Zusatzstoffe, vorzugsweise 0,1 - 58 Gew-%, insbesondere 0,1 - 50 Gew-%.

Es wurde überraschend gefunden, daß der Zusatz von Lanosterin zu üblichen kosmetischen ölen feste, wachsartige Produkte ergibt, die je nach Anteil von Lanosterin und Art des öles einen Tropfpunkt von höher als 60 °C erreichen und sich somit ausgezeichnet als Grundlage für kosmetische Stifte oder andere feste kosmetische Zusammensetzungen eignen.

Lanosterin ist ein steroider Alkohol, Lanosta-8,24-dien-3β-ol bzw. 4,4,14α-Trimethyl-5α-cholesta-8,24-dien-3β-ol, der weitgehend aus Lanolin erhalten wird, z.B. durch fraktionierte Kristallisation. Lanolin wiederum wird aus Wollwachs von Schafen gewonnen und ist in der Kosmetik als Salbengrundlage bekannt. Nähere Einzelheiten über die Aufklärung der zu Lanosterin führenden Trennverfahren sind in der Monografie "Woolwax" von E.V. Truter, 1956, Intersience, angegeben, auf die hier Bezug genommen wird. Lanosterin hat ein Molekulargewicht von 426,7, bildet farblose Kristalle, hat einen Schmelzpunkt von 138-140 °C und ist löslich in Chloroform und Ethanol.

Als Handelsprodukt ist Lanosterin unter der Bezeichnung LANOSTEROL von verschiedenen Lieferanten erhältlich, beispielsweise von Croda Chemicals Ltd., East Yorkshire, England, Die Begriffe Lanosterin und Lanosterol werden hier synonym verwendet.

Lanosterin hat wegen seiner Verwandtschaft mit Cholesterin Bedeutung bei der Therapie von Stoffwechselerkrankungen und ist darüber hinaus als Emulgator bekannt.

Insofern gab es für den Fachmann keinerlei Hinweis, daß die Kombination von kosmetischen ölen und Lanosterin sowie gegebenenfalls weiterhin die Festigkeit und die kosmetischen Eigenschaften verbessernden Kautschuken, Copolymeren und Polymeren zu festen Produkten, ähnlich den klassischen Lippenstiften, jedoch mit Transparenz, führen könnte.

Die für die Erfindung eingesetzten öle können übliche kosmetische öle sein, wie ein Mineralöl; hydriertes Polyisobuten (INCI-Name: Hydrogenated polyisobutene); synthetisches oder aus Naturprodukten hergestelltes Squalan (INCI-Name: Squalane, z.B. Synthesqual® , Cosbiol® ); kosmetische Ester oder Ether, die verzweigt oder unverzweigt, gesättigt oder ungesättigt sein können; pflanzliche öle; oder Gemische zweier oder mehrerer davon.

Als Ester oder Ether sind zum Beispiel geeignet (INCI-Na-men): Dipentaerythrityl hexacaprilate/hexacaprate/tridecyl trimellitate/tridecyl stearate/neopentyl glycol dicaprylate dicaprate, Propylene glycol dioctanoate 5, Propylene glycol dicaprylate 2,30 dicaprate, Tridecyl stearate/neopentyl glycol dicaprylate dicaprate/tridecyl trimellitate, Neopentyl glycol dioctanoate, Isopropyle myristate, Diisopropyl dimer dilinoleate, Trimethylpropane triisostearate, Myristyl ether, Stearyl ether, Butyl ether, Dicaprylyl ether, PPG1-PEG9 Lauroyl glycol ether, PPG15 Stearyl ether, PPG14 Butyl ether, Fomblin HC25.

Besonders geeignet öle sind beispielsweise Mineralöle, Hydrogenated Polyisobuten, Polyisopren, Squalane, Tridecyltrimellitat, Trimethylpropan-triisostearat, Isodecylcitrat, Neopentylglycol-diheptanoat, PPG-15-stearylether, Calendulaöl, Jojobaöl, Avocadoöl, Macadamianußöl oder ein Gemisch mehrerer davon. Je nachdem welche öle ausgewählt werden, werden die kosmetischen Eigenschaften der festen Zusammensetzung beeinflußt, wie Transparenzgrad, Weichheit, Härte, Spreitungswirkung. So erhält man beispielsweise mit 50 bis 70 Gew-% Squalane® oder Hydrogenated Polyisobuten eine völlig transparente feste Zusammensetzung.

Für die Erfindung nützliche kosmetische Wirkstoffe können sein Polymere, Copolymere, Siliciumdioxid (Silica), Vitamine, organische öllösliche Farbstoffe, anorganische Pigmente, Antioxidationsmittel, Deodorants, Sonnenschutzmittel, Duftstoffe und weitere aus der Kosmetik bekannte Wirkstoffe, wie Kaolin oder mit SiO₂ gemäß WO96/17588 modifiziertes Kaolin.

Die erfindungsgemäße Zusammensetzung, bestehend allein aus einem öl und Lanosterin führt bereits zu einer festen kosmetischen Zusammensetzung mit befriedigenden Eigenschaften hinsichtlich der Festigkeit, auch für kosmetische Stifte. Eine Verbesserung dieser Festigkeitseigenschaften sowie der sonstigen kosmetischen Eigenschaften ist möglich durch die zuvor und nachfolgend genannten Wirk- und Zusatzstoffe.

Zur Verbesserung der Festigkeit und Stabilität der erfindungsgemäßen Zusammensetzungen, insbesondere in Form von kosmetischen Stiften, dient der Gehalt von Polymeren oder Copolymeren, wie beispielsweise von hydrierten Styren/Methylstyren/Inden-Copolymeren, z.B. Régalite R101® von Hercules; Copolymeren von Vinylpyrrolidon und langkettigen α-Olefinen, wie Antaron V220® , Antaron V216® , Unimer U15® ; Salzen von Fettsäureestern, wie Sodium Isostearoyl lactylate (Pationic ISL® von Rita Corp.); und PEG-120 Methyl glucose dioleate (z.B. Glucamate DOE 120® ); sowie Gemischen davon, sowie von Kautschuken, wie Polyisopren. Polyisopren ist besonders geeignet.

Besonders bevorzugt sind hydrierte Styren/Methylstyren/-Inden-Copolymere, da sie die Bruchfestigkeit von Stiften mindern ohne eine Trübung in die Transparenz des Stiftes einzubringen. Außerdem gewährleisten hydrierte Kohlenwasserstoffharze eine bessere Formbarkeit und Einfüllbarkeit der Zusammensetzung in Lippenstift- oder dünne Stiftformen.

Eine bevorzugte erfindungsgemäße Zusammensetzung enthält 40 bis 95 Gew-% eines kosmetisch annehmbaren öles oder ölgemisches, 1 bis 10 Gew-% Lanosterin und 5 bis 30 Gew-% eines oder mehrerer kosmetischer Copolymere oder Polymere sowie geeignete Duft- oder Farbstoffe oder Gemische davon.

Eine weitere Verfestigung der Zusammensetzung kann auch durch den Zusatz anorganischer Stoffe, wie Silica (Siliciumdioxid), z.B Aerosil® , Cab-O-Sil® , Syloid® , oder durch Pigmente, Kaolin, mit SiO₂ modifiziertes Kaolin, TiO₂ usw. erfolgen. über darin gegebenenfalls enthaltene Hydroxygruppen können Bindungen zur Verfestigung des Produktes ausgebildet werden, wobei die Transparenz nicht beeinträchtigt wird und ein eventuelles öliges Feeling des Stiftes auf der Haut herabgesetzt werden kann.

Weitere Zusatz- bzw. Wirkstoffe in den kosmetischen Zusammensetzungen können sein Vitamine, z.B. Vitamin A oder Vitamin A-Derivate; gefärbte Pflanzenextrakte, wie fettlöslicher Gardenienextrakt, fettlöslicher Karottenextrakt, Paprika-LS-Extrakt, β-Caroten, Lithospermum-Extrakt; organische Lichtschutzmittel wie z.B. Octylmethoxycinnamate; Methyl gluceth 10 oder Methyl gluceth 20; sowie aktive Deodorantien, wie Triclosan, oder Geruchslöscher, wie Grillocin® .

Von besonderem Interesse ist die Zugabe von Duftstoffen. Die erfindungsgemäße feste Zusammensetzung weist den großen Vorteil auf, daß in ihr hohe Anteile von Parfümen, meist gelöst in Alkoholen und als Konzentrat vorliegend, eingearbeitet werden können, ohne daß die Zusammensetzung undurchsichtig wird. Auf diese Weise lassen sich Parfümanteile von 0,1 bis 15 Gew-%, bezogen auf das Gesamtgewicht der Zusammensetzung, in diese aufnehmen. Ein hergestellter transparenter Duftstift gestattet eine besonders ästhetische und zugleich leichte Applikation z.B. im Hals- und Gesichtsbereich, in Achselhöhlen usw.

Weiterhin von besonderem Interesse ist die Zugabe von Farbstoffen und Pigmenten zu den erfindungsgemäßen Zusammensetzungen. Es können alle bekannten organischen Farbstoffe und anorganischen Pigmente verwendet werden, die in der Kosmetik üblich sind. Dabei ist zu beachten, daß zur Beibehaltung der Transparenz einer Zusammensetzung die Einfärbung durch organische öllösliche Farbstoffe erforderlich ist, während für transluzente oder noch stärker eingetrübte Zusammensetzungen auch anorganische Pigmente verwendet werden können.

Die organischen öllöslichen Farbstoffe können der Zusammensetzung problemlos hinzugefügt werden. Die Verarbeitung mit anorganischen Pigmenten erfolgt vorteilhaft in der Weise, daß das Pigment oder Pigmentgemisch mit einem öl vermahlen wird und dann der Zusammensetzung hinzugesetzt wird. Geringe Mengen Pigmente, etwa im Bereich von 0,1 bis 0,3 Gew-%, führen zu farbigen, nahezu transparenten festen Zusammensetzungen. Bei größeren Pigmentmengen, etwa bei 3 bis 4 Gew-%, ist die Zusammensetzung trübe oder undurchsichtig. Daher ist auch die Formulierung von Lippenstiften, Make-up-Stiften, Lippenglanz, Rouge oder Grundierungen möglich, was ebenfalls besondere Ausführungsformen der Erfindung darstellen, bei denen die Pigment- gehalte bis zu 8 Gew-% betragen können.

Pigmente, Pigmentgemische oder Pulver mit pigmentartiger Wirkung, worunter auch solche mit Perlglanz-Effekt zu verstehen sind, können zum Beispiel umfassen Eisenoxide, Titan(di)oxid, Glimmer, Kaolin, Talkum, Glimmer-Titanoxid, Glimmer-Titanoxid-Eisenoxid, Wismutoxychlorid, Nylonkügelchen, Keramikkügelchen, expandierte und nichtexpandierte synthetische Polymerpulver, pulverförmige natürliche organische Verbindungen wie gemahlene Festalgen, verkapselte und unverkapselte Getreidestärken sowie Glimmer-Titanoxid-organischer Farbstoff.

Selbstverständlich können auch erfindungsgemäße feste Zusammensetzungen mit in erster Linie Duftkompomenten , z.B. Duftstifte, mit Pigmenten versehen werden, und umgekehrt können mehr oder weniger pigmentierte Zusammensetzungen mit bestimmten Duftanteilen versehen werden.

Es können auch Flüssigkristalle hinzugesetzt werden z.B. Licritherm® von Merck.

Ein weiteres besonderes Merkmal der Erfindung besteht darin, daß die Zusammensetzung wasserphasenfrei ist, d.h. sie enthält kein gesondert hinzugegebenes Wasser, das als eigenständige Phase aufzufassen wäre. Die Zusammensetzung enthält höchstens solche geringen Wassermengen, die physikalisch gebunden durch einzelne Zuschlagstoffe eingetragen werden. Dieser Anteil liegt jedoch deutlich unter 5 Gew-%.

Die Herstellung der erfindungsgemäßen festen Zusammensetzungen erfolgt allgemein in der Weise, daß das öl mit Lanosterin bei erhöhter Temperatur von etwa 40 bis 90 °C unter Rühren vermischt wird. Die Temperatur kann gegebenenfalls auch höher sein, wenn harzartige Polymere und Copolymere als Zusatzstoffe hinzugegeben werden und deren Schmelzpunkt höher liegt. Es ist vorteilhaft, derartige Stoffe durch Schmelzen in die Zusammensetzung aufzunehmen. Danach werden weitere kosmetische Wirkstoffe oder sonstige Zusatzstoffe unter Rühren und bei Temperaturen, die diesen Stoffen angemessen sind und dem Fachmann auf diesem Gebiet bekannt sind, hinzugegeben. Bei Temperaturen zwischen etwa 60 und 70 °C wird das homogen Gemisch, das zuvor durch langsames Rühren entlüftet worden ist, in entsprechende Formen gegossen und abgekühlt.

Bevorzugte Ausführungsformen der Erfindung sind
1) feste Zusammensetzungen in Form von transparenten festen Make-up's, gefärbt oder ungefärbt, mit Duftstoffen oder ohne Duftstoffe, mit oder ohne Aktivstoffe, Sonnenschutzfilter oder Flüssigkristalle;
2) transparente Duftkompositionen, gefärbt oder ungefärbt, mit oder ohne Aktivstoffe, Sonnenschutzfilter oder Flüssigkristalle;
3) transparente Sonnenschutzkompositionen, gefärbt oder ungefärbt, mit Duftstoffen oder ohne Duftstoffe, mit oder ohne Aktivstoffe oder Flüssigkristalle.
4) transparente Deodorant- und/oder Antitransprirantkompositionen, gefärbt oder ungefärbt, mit Duftstoffen, mit oder ohne Aktivstoffe oder Flüssigkristalle.

Die Erfindung soll nachstehend durch Beispiele näher erläutert werden. Alle Prozentangaben sind Gewichtsprozente, sofern nicht anderes angegeben ist.

### Beispiel 1

### Ungefärbter transparenter Stift (1)

Die Herstellung erfolgte durch Vermischen aller Bestandteile bei einer Temperatur bis etwa 115 °C. Nach Schmelzen des Wachses wurde das homogene Produkt auf 70 °C abgekühlt und in Formen gegossen.

| | |
|---|---|
| Lanosterin | 3 |
| Squalane® | q.s. |
| Tridecyl trimellitate | 15 |
| Hydrogenated styrene/Methylstyrene/Indene Copolymer | 15 |

### Beispiel 2

### Ungefärbter transparenter Stift (2)

Die Herstellung erfolgte wie im Beispiel 1.

| | |
|---|---|
| Lanosterin | 8 |
| Hydrogenated polyisobuten | q.s. |
| Tridecyl trimellitate | 25 |
| Hydrogenated styrene/Methylstyrene/Indene Copolymer | 22 |

### Beispiel 3

### Gefärbter transparenter Stift

| | |
|---|---|
| Lanosterin | 6 |
| Hydrogenated polyisobuten | q.s. |
| Tridecyl trimellitate | 24,97 |
| Hydrogenated styrene/Methylstyrene/Indene Copolymer | 8 |
| Red 7 Ca⁺⁺ Lake (CI 15850:1) | 0,02 |
| Red 33 Al Lake (CI 17200) | 0,01 |

Lanosterin, das Copolymere, Tridecyl trimellitate und Hydrogenated polyisobuten wurden unter Rühren mit ca. 300 U/Min. bei Temperaturen bis etwa 115 °C geschmolzen. Nach Abschluß des Schmelzvorganges wurde die Temperatur auf 95 °C abgesenkt und die getrennt mit Trimellitat vorgemischten Pigmente hinzugegeben. Unter Rühren mit ca. 350 U/Min. wurde Silica hinzugesetzt. Nach Einführung von Silica wurde unter weiterer Absenkung der Temperatur langsam gerührt, um das homogene Gemisch zu entlüften. Bei 65 °C wurde das Produkt in Formen gegossen.

### Beispiel 4

### Transparenter Stift mit farbigem Pflanzenextrakt

| | |
|---|---|
| Lanosterin | 7 |
| Hydrogenated polyisobuten | 53 |
| Tridecyl trimellitate | 24,90 |
| Hydrogenated styrene/Methylstyrene/Indene Copolymer | 10 |
| Silica | 4 |
| Lithospermum-Extrakt | 0,10 |

Es wurde wie im Beispiel 3 gearbeitet. Der Lithospermum-Extrakt wurde bei 80 °C nach der Einarbeitung von Silica hinzugegeben.

### Beispiel 5

### Transparenter Stift mit Lichtschutz

| | |
|---|---|
| Lanosterin | 8 |
| Hydrogenated polyisobuten | q.s |
| Trimethylolpropane triisostearate | 10 |
| Hydrogenated styrene/Methylstyrene/Indene Copolymer | 10 |
| Octylmethoxycinnamate | 6 |

Es wurde in gleicher Weise wie im Beispiel 1 verfahren. Das Octylmethoxycinnamate wurde bei 70 °C während der Abkühlung hinzugegeben. Der Lichtschutzfaktor (SPF) betrug 8.

### Beispiel 6

### Transparenter Stift mit Deodorant-Eigenschaften

| | |
|---|---|
| Lanosterin | 5,5 |
| Polyisopren | q.s. |
| Triclosan | 0,3 |
| Squalane | 3 |
| Fragance | 1 |

Es wurde in ähnlicher Weise wie im Beispiel 1 verfahren. Das Triclosan wurde bei 70 °C während der Abkühlung hinzugegeben und die Duftstoffe unmittelbar vor dem Eingießen in Formen (bei etwa 60 °C).

### Beispiel 7

### Duftstift

| | |
|---|---|
| Lanosterin | 6,9 |
| Hydrogenated polyisobuten | 65 |
| Tridecyl trimellitate | 5 |
| Hydrogenated styrene/Methylstyrene/Indene Copolymer | 10 |
| Silica | 3,5 |
| Fragance | 10 |

Es wurde wie im Beispiel 3 gearbeitet. Die Duftstoffe wurden bei 60 °C unmittelbar vor dem Einfüllen in die Formen hinzugegeben.

### Beispiel 8

### Make-up-Stift

| | |
|---|---|
| Lanosterin | 5 |
| Hydrogenated polyisobuten | q.s. |
| Tridecyl styrene | 23,5 |
| Methylstyrene/Indene Silica | 8,5 |
| Silica | 2 |
| Rotpigment | 0,5 |
| Schwarzpigment | 0,2 |
| Kaolin modifiz. nach WO96/17588 | 1,2 |
| TiO₂ | 1,0 |
| Braunpigment | 0,8 |

Es wurde ähnlich wie im Beispiel 3 gearbeitet.

## Patentansprüche

1. Feste kosmetische Zusammensetzung auf Basis verfestigter öle, **dadurch gekennzeichnet, daß** sie 40 bis 95 Gew-% eines kosmetisch annehmbaren öles oder ölgemisches und 1 bis 10 Gew-% Lanosterin enthält und transparent ist, wobei die Anteile jeweils bezogen sind auf die Gesamtmenge der Zusammensetzung .

2. Zusammensetzung nach Anspruch 1, **dadurch gekennzeichnet, daß** das öl aus der Gruppe ausgewählt ist, die aus Mineralöl, Hydrogenated polyisobuten, Polyisopren, Squalane, pflanzlichen ölen, kosmetischen Estern, kosmetischen Ethern und Gemischen davon besteht.

3. Zusammensetzung nach Anspruch 2 , **dadurch gekennzeichnet, daß** das öl Hydrogenated polyisobuten, Polyisopren, Squalane, Tridecyltrimelltrimellitat, Trimethylpropan-triisostearat, Isodecylcitrat, Neopentylglycol-diheptanoat, PPG-15-stearylether, Calendulaöl, Jojobaöl, Avocadoöl, Macadamianußöl oder ein Gemisch mehrerer davon ist.

4. Zusammensetzung nach Anspruch 1 , **dadurch gekennzeichnet, daß** sie 40 bis 95 Gew-% eines kosmetisch annehmbaren öles oder ölgemisches, 1 bis 10 Gew-% Lanosterin und 5 bis 30 Gew-% eines oder mehrerer kosmetischer Copolymere oder Polymere enthält.

5. Zusammensetzung nach Anspruch 1, **dadurch gekennzeichnet, daß** sie 0,1 bis 58 Gew-% eines oder mehrerer kosmetischer Wirkstoffe oder Zusatzstoffe umfaßt, die ausgewählt sind unter Polymeren, Copolymeren, Silica, Vitaminen, organischen öllöslichen Farbstoffen, anorganischen Pigmenten, Antioxidationsmitteln, Deodorants, Sonnenschutzmitteln, Duftstoffen, Kaolin, mit SiO₂ modifiziertem Kaolin, gefärbten Pflanzenextrakten, organischen Lichtschutzmitteln, aktive Deodorantien, Flüssigkristallen und Gemischen davon; wobei die Anteile jeweils bezogen sind auf die Gesamtmenge der Zusammensetzung; und wobei die Zusammensetzung frei von einer Wasserphase ist.

6. Zusammensetzung nach Anspruch 5 , **dadurch gekennzeichnet, daß** sie 0,1 bis 50 Gew-% eines oder mehrerer kosmetischer Wirkstoffe oder Zusatzstoffe umfaßt.

7. Zusammensetzung nach Anspruch 5, **dadurch gekennzeichnet, daß** die Polymeren und Copolymeren aus der Gruppe ausgewählt sind, die aus hydrierten Styren/Methylstyren/Inden-Copolymeren, Copolymeren von Vinylpyrrolidon und langkettigen α-Olefinen, Salzen von Fettsäureestern, PEG-120 Methyl glucose dioleate und Gemischen davon besteht.

8. Zusammensetzung nach Anspruch 5, **dadurch gekennzeichnet, daß** sie transluzent ist.

9. Zusammensetzung nach einem der Ansprüche 1 bis 8 , **dadurch gekennzeichnet,** daß sie in Form von kosmetischen Stiften mit Durchmessern von 0,5 bis 5 cm vorliegt.

10. Zusammensetzung nach Anspruch 1 , **dadurch gekennzeichnet, daß** sie als kosmetischer wasserphasenfreier, transparenter Stift mit Duftstoffen vorliegt.

11. Zusammensetzung nach Anspruch 9 oder 10, **dadurch gekennzeichnet, daß** der Stift gefärbt oder pigmentiert ist.

12. Zusammensetzung nach einem der Ansprüche 1 bis 9 , **dadurch gekennzeichnet, daß** sie in Form einer Make-up-Zubereitung vorliegt.

13. Zusammensetzung nach einem der Ansprüche 1 bis 7 , **dadurch gekennzeichnet, daß** sie in Form einer Sonnenschutz-Zubereitung vorliegt.

## Claims

1. A solid cosmetic composition on the basis of solidified oils, which comprises 40 to 95% by weight of a cosmetically acceptable oil or oil mixture and 1 to 10% by weight of lanosterol, each based on the total amount of the composition, and which composition is transparent.

2. Composition according to Claim 1, wherein the oil is selected from the group consisting of mineral oil, hydrogenated polyisobutene, polyisoprene, squalane, vegetable oils, cosmetic esters, cosmetic ethers and mixtures thereof.

3. Composition according to Claim 2, wherein the oil is hydrogenated polyisobuten, polyisoprene, squalane, tridecyl trimellitate, trimethylpropane triisotearate, isodecyl citrate, neopentyl glycol diheptanoate, PPG15 stearyl ether, calendula oil, jojoba oil, avocado oil, macadamia-nut oil or a mixture thereof.

4. Composition according to Claim 1, comprising 40 to 95% by weight of a cosmetically acceptable oil or oil mixture, 1 to 10% by weight of lanosterol and 5 to 30% by weight of one or more cosmetic polymers or copolymers.

5. Composition according to Claim 1, comprising 0.1 to 58% by weight of one or more active cosmetic ingredients or additives which are selected from the group consisting of polymers, copolymers, silica, vitamins, organic oil-soluble colorants, inorganic pigments, antioxidants, deodorants, sunscreens, fragrances, kaolin, with SiO₂ modified kaolin, colored plant extracts, organic sunscreens, active deodorants, liquid crystals and mixtures thereof, the percentage is based in each case on the total weight of the composition, and wherein the composition is free of a watery phase.

6. Composition according to Claim 5, comprising 0.1 to 50% by weight of one or more active cosmetic ingredients or additives.

7. Composition according to Claim 5, wherein polymers and copolymers are selected from the group consisting of hydrogenated styrene/methyl styrene/indene copolymers, copolymers of vinyl pyrrolidone and long-chain α-olefins, salts of fatty acid esters, PEG-120 methyl glucose dioleate and mixtures thereof.

8. Composition according to Claim 5, wherein said composition is translucent.

9. Composition according to one of the Claims 1 to 8, wherein said composition is present in the form of cosmetic sticks with a diameter of 0.5 to 5 cm.

10. Composition according to Claim 10, wherein said composition is present in the form of a transparent cosmetic stick with fragrances and without an aqueous phase.

11. Composition according to Claims 9 or 10, wherein said stick is colored or pigmented.

12. Composition according to one of the Claims 1 to 9, wherein said composition is present in the form of a makeup formulation.

13. Composition according to one of the Claims 1 to 7, wherein said composition is present in the form of a sunscreen formulation.

## Revendications

1. Composition cosmétique solide à base d'huiles solidifiées, **caractérisée en ce qu'**elle contient de 40 à 95 % massiques d'une huile ou d'un mélange d'huile cosmétiquement acceptable et de 1 à 10 % massiques de lanostérine, les pourcentages étant chacun rapportés sur la quantité totale de la composition.

2. Composition selon la revendication 1, **caractérisée en ce que** l'huile est choisie parmi le groupe constitué de l'huile minérale, de hydrogenated polyisobuten, polyisopren, squalane, des huiles végétales, des esters cosmétiques, d'éthers cosmétiques et de mélanges de ceux-ci.

3. Composition selon la revendication 2, **caractérisée en ce que** l'huile est le hydrogenated polyisobuten, le polyisopren, le squalane, le tridecyltrimellitate, le trimethylpropane triisostearate, l'isodecylcitrate, le neopentyl glycol diheptanoate, PPG-15-stearylether, l'huile de Calendula, l'huile de Jojoba, l'huile d'Avocado, l'huile de noix Macadamia ou un mélange de plusieurs d'entre elles.

4. Composition selon la revendication 1, **caractérisée en ce qu'**elle contient de 40 à 95 % massiques d'une huile ou d'un mélange cosmétiquement acceptable, de 1 à 10 % massiques de lanostérine et de 5 à 30 % massiques d'un ou de plusieurs copolymères ou polymères cosmétiques.

5. Composition selon la revendication 1, **caractérisée en ce qu'**elle comprend de 0,1 à 85 % massiques d'un ou de plusieurs principes actifs choisis parmi les polymères, copolymères, la silice, les vitamines, les colorants organiques solubles dans l'huile, les pigments inorganiques, les antioxydants, les déodorants, les protecteurs d'UV, les parfums, le kaolin, le kaolin modifié au SiO₂, les extraits végétaux teintés, les protecteurs d'UV organiques, les déodorants actifs, les cristaux liquides et des mélanges de ceux-ci ; les pourcentages étant à chaque fois rapportés sur la quantité totale de la composition ; et la composition étant dépourvue de phase aqueuse.

6. Composition selon la revendication 5, **caractérisée en ce qu'**elle comprend de 0,1 à 50 % massiques d'un ou plusieurs principes actifs ou additifs.

7. Composition selon la revendication 5, **caractérisée en ce que** les polymères et copolymères sont choisis parmi le groupe constitué des copolymères styrène/méthylstyrène/indène, copolymères de vinylpyrolidone et des α-oléfines à longues chaînes, des sels d'esters d'acides gras, de PEG-120 Methyl glucose dioleate et des mélanges de ceux-ci.

8. Composition selon la revendication 5, **caractérisée en ce qu'**elle est translucide.

9. Composition selon l'une des revendications 1 à 8, **caractérisée en ce qu'**elle se présente sous forme de bâtons cosmétiques avec des diamètres de 0,5 à 5 cm.

10. Composition selon la revendication 1, **caractérisée en ce qu'**elle se présente sous forme de bâton cosmétique transparent, dépourvu de phase aqueuse et doté de parfum.

11. Composition selon la revendication 9 ou 10, **caractérisée en ce que** le bâton est teinté ou pigmenté.

12. Composition selon l'une des revendications 1 à 9, **caractérisée en ce qu'**elle se présente sous forme de préparation de maquillage.

13. Composition selon l'une des revendications 1 à 7, **caractérisée en ce qu'**elle se présente sous forme de préparation à protection d'UV.
